# FASCICULE DE BREVET EUROPEEN

(11) **EP 0 456 023 B1**
(45) Date de publication et mention de la délivrance du brevet: **15.06.1994**
(21) Numéro de dépôt: 91106385.7
(22) Date de dépôt: 20.04.1991
(51) Int. Cl.: C07D 311/62

(54) **Procédé d'obtention de complexes de catechines**
Verfahren zur Herstellung von Pyrokatechinkomplexen
Process for preparation of catechol complexes

(30) Priorité: 09.05.1990 CH 1573/90
(43) Date de publication de la demande: 13.11.1991
(73) Titulaire: SOCIETE DES PRODUITS NESTLE S.A., CH-1800 Vevey (CH)
(72) Inventeur: Lunder, Tito Livio, CH-1110 Morges (CH)

(56) Documents cités:
- FR-A- 1 584 725
- DERWENT JAPANESE PATENT REPORTS, vol. 13, no. 3 (C-557)[3351], 6 janvier 1989,Derwent Publications Ltd, Londres, GB; & JP-A-63 214 183 (MITSUI NORIN K.K.) 06-09-1988

## Description

L'invention concerne un procédé d'obtention de complexes de catéchines.

Jusqu'à présent, les procédés d'obtention de catéchines à partir de thé ou d'autres plantes impliquaient l'utilisation de solvants chauds qui augmentaient les risques de polymérisation. Les catéchines peuvent aussi être extraites à froid mais les tannins condensés doivent être éliminés en saturant la phase aqueuse avec du chlorure de sodium.

Le procédé, objet de la présente invention, permet d'effectuer une bonne extraction des catéchines contenues dans certaines plantes, tout en éliminant les tannins polymérisés.

L'invention concerne un procédé d'obtention de complexes de catéchines, dans lequel on infuse des feuilles de thé vert ou toute autre plante contenant des catéchines, on concentre l'extrait jusqu'à une consistance de liqueur, on extrait cette liqueur au dichlorométhane pour éliminer les pigments, on mélange la phase aqueuse avec du sable de mer purifié pour former une pâte, on élue les complexes de catéchines avec de l'acétone et on évapore l'acétone pour obtenir une poudre. De manière avantageuse, on élue les catéchines avec de l'acétone contenant une faible quantité d'eau.

Par complexes de catéchines on entend notamment la catéchine, l'épicatéchine, le catéchine gallate et l'épigallotechingallate.

Les catéchines trouvent une application notamment comme additifs dans des infusions de thé noir pendant la fabrication de thé noir instantané pour éviter la formation de crème de thé, qui est un complexe de caféine et de polyphénols. On sait aussi que les catéchines sont utilisables comme sources de vitamines P et peuvent entrer dans des spécialités pharmaceutiques pour la prévention de la fragilité capillaire.

Comme autre plante contenant des complexes de catéchines, on peut citer notamment la vigne rouge.

On a constaté, de manière surprenante, selon l'invention, que si on utilise du sable de mer purifié, les tannins polymérisés sont fortement retenus sur ledit sable, tandis que les catéchines sont aisément éluées avec de l'acétone, contenant avantageusement une faible quantité d'eau.

Les feuilles de thé sont infusés par charge ou à contre-courant, soit à chaud à une température comprise entre 90 et 130°C à une pression comprise entre 1 et 3 bars, de préférence 2 bars, pendant 10 à 30 minutes, soit à froid à une température comprise entre 20 et 30°C à pression atmosphérique pendant 5 à 6 heures. Le traitement à froid permet de ménager le produit et d'éviter l'oxydation des catéchines et le traitement à chaud permet d'augmenter le rendement d'extraction.

Après infusion, les feuilles sont séparées par centrifugation et l'infusion est concentrée jusqu'à une liqueur lourde ayant une teneur en matière sèche comprise entre 25 et 30%, selon des techniques conventionnelles.

Les pigments sont éliminés par une extraction liquide-liquide avec un solvant tel que le dichlorométhane. Lors de cette extraction le rapport en volume liqueur:solvant est compris entre 1:5 et 1:20.

La phase aqueuse est ensuite mélangée avec du sable de mer purifié dans un percolateur, dans une colonne ou dans tout autre dispositif permettant l'évaporation subséquente d'eau. L'élution des catéchines se fait avec de l'acétone contenant une faible quantité d'eau. On verse l'acétone sur le mélange de sable de mer et de thé et on recycle plusieurs fois cette acétone pour garantir une élution complète des complexes de catéchines.

On chasse ensuite l'acétone sous pression réduite et on poursuit le séchage jusqu'à l'obtention d'une poudre jaunâtre. La solution aqueuse obtenue après élimination de l'acétone peut être aussi séchée par pulvérisation ou par lyophilisation.

Le rendement du procédé selon l'invention est d'environ 60 à 70%.

La suite de la description est faite en référence aux exemples.

### Exemple 1

100 g de feuilles de thé vert sec sont infusés dans de l'eau désionisée à 95°C pendant 10 minutes.

Les feuilles épuisées sont séparées par centrifugation et l'infusion claire est concentrée sous pression réduite jusqu'à la consistance d'une liqueur lourde ayant une teneur en matière sèche d'environ 27 à 30%.

Cette liqueur lourde est extraite au dichlorométhane pour éliminer les pigments du thé vert.

La phase aqueuse est mélangée avec une quantité de sable de mer purifié suffisante pour donner une pâte qu'on sèche à 70°C sous vide. Les catéchines sont éluées avec 200 ml d'acétone contenant 5% d'eau. On relave le mélange thé:sable avec 100 ml d'acétone contenant 5% d'eau.

La phase organique est évaporée sous pression réduite et on poursuit la concentration de la phase aqueuse jusqu'à l'obtention d'un résidu de consistance sirupeuse qui est séché par atomisation. Alternativement, après avoir éliminé l'acétone, la phase aqueuse peut être lyophilisée. La poudre ainsi obtenue est soluble dans l'eau et l'éthanol.

### Exemple 2

100 ml de feuilles de thé vert sec sont extraits dans une cellule avec de l'eau désionisée à 130-125°C sous une pression de 2 bars pendant 15 min. en recyclant continuellement l'extrait.

Les feuilles épuisées sont lavées avec de l'eau fraîche qui est ajoutée à l'extrait précédent. La centrifugation ou filtration sont généralement inutiles car les couches de feuilles ainsi que les souches sur le bas et le haut des opercules agissent comme des filtres efficaces.

L'extrait est évaporé jusqu'à une teneur en matière sèche d'environ 20-25% et on fait une extraction au dichlorométhane pour éliminer les pigments.

La phase aqueuse visqueuse est mélangée avec une quantité suffisante de sable de mer purifié pour donner une pâte qu'on sèche à 60°C sous vide.

Les catéchines sont retenues sur le sable et éluées ensuite avec de l'acétone contenant 5% d'eau.

La phase organique est évaporée sous pression réduite et on poursuit la concentration de la phase aqueuse jusqu'à l'obtention d'un résidu de consistance sirupeuse qui est séché par atomisation. Alternativement, après avoir éliminé l'acétone, la phase aqueuse peut être lyophilisée. La poudre ainsi obtenue est soluble dans l'eau et l'éthanol.

## Revendications

1. Procédé d'obtention de complexes de catéchines caractérisé en ce qu'on infuse des feuilles de thé vert dans l'eau, on concentre l'extrait jusqu'à une consistance de liqueur, on extrait cette liqueur au dichlorométhane pour éliminer les pigments, on mélange la phase aqueuse avec du sable de mer purifié pour former une pâte, on élue les complexes de catéchines avec de l'acétone et on évapore l'acétone pour obtenir une poudre.

2. Procédé selon la revendication 1, caractérisé en ce qu'on infuse les feuilles de thé vert à une température comprise entre 90 et 130°C à une pression comprise entre 1 et 3 bars pendant 10 à 30 min.

3. Procédé selon la revendication 1, caractérisé en ce qu'on infuse les feuilles de thé vert à une température comprise entre 20 et 30°C à pression atmosphérique pendant 5 à 6 h.

4. Procédé selon l'une des revendications 1 à 3, caractérisé en ce que l'on concentre l'extrait jusqu'à obtenir une teneur en matière sèche comprise entre 25 et 30%.

5. Procédé selon l'une quelconque des revendications 1 à 3, caractérisé en ce que l'on extrait la liqueur avec du dichlorométhane dans un rapport en volume liqueur:dichlorométhane compris entre 1:5 et 1:20.

## Patentansprüche

1. Verfahren zur Herstellung von Catechinkomplexen, dadurch gekennzeichnet, daß Grünteeblätter in Wasser infundiert werden, der Extrakt bis zu einer Likörkonsistenz konzentriert wird, dieser Likör zur Beseitigung der Pigmente mit Dichlormethan extrahiert wird, die wässrige Phase zur Bildung eines Breis mit gereinigtem Meersand gemischt wird, die Catechinkomplexe mit Aceton eluiert werden und das Aceton abgedampft wird, um ein Pulver zu erhalten.

2. Verfahren nach Anspruch 1, dadurch gekennzeichnet, daß die Grünteeblätter bei einer Temperatur von 90 bis 130°C und einem Druck von 1 bis 3 bar 10 bis 30 Minuten lang infundiert werden.

3. Verfahren nach Anspruch 1, dadurch gekennzeichnet, daß die Grünteeblätter bei einer Temperatur von 20 bis 30°C und atmosphärischem Druck 5 bis 6 Stunden lang infundiert werden.

4. Verfahren nach einem der Ansprüche 1 bis 3, dadurch gekennzeichnet, daß der Extrakt konzentriert wird, bis man einen Trockensubstanzgehalt von 25 bis 30 % erhält.

5. Verfahren nach einem der Ansprüche 1 bis 3, dadurch gekennzeichnet, daß der Likör mit Dichlormethan in einem Volumensverhältnis von Likör zu Dichlormethan von 1:5 bis 1:20 extrahiert wird.

## Claims

1. A process for obtaining catechol complexes, characterized in that leaves of green tea are infused in water, the extract is concentrated to the consistency of a liquor, the liquor is extracted with dichloromethane to eliminate the pigments, the aqueous phase is mixed with purified sea sand to form a paste, the catechol complexes are eluted with acetone and the acetone is evaporated to obtain a powder.

2. A process as claimed in claim 1, characterized in that the leaves of green tea are infused at a temperature of 90 to 130°C under a pressure of 1 to 3 bar over a period of 10 to 30 minutes.

3. A process as claimed in claim 1, characterized in that the leaves of green tea are infused at a temperature of 20 to 30°C under atmospheric pressure over a period of 5 to 6 hours.

4. A process as claimed in any of claims 1 to 3, characterized in that the extract is concentrated to a dry matter content of 25 to 30%.

5. A process as claimed in any of claims 1 to 3, characterized in that the liquor is extracted with dichloromethane in a ratio by volume of liquor to dichloromethane of 1:5 to 1:20.
